# EUROPEAN PATENT SPECIFICATION

(11) **EP 3 298 025 B1**
(45) Date of publication and mention of the grant of the patent: **11.02.2026**
(21) Application number: 16730260.3
(22) Date of filing: 17.05.2016
(51) Int. Cl.: C07K 7/08, A61K 38/10, C07K 14/46

(54) **NEW AMINO ACID SEQUENCES WITH MICROBICIDAL ACTIVITY DERIVED FROM NAJA ATRA CARDIOTOXIN 1 (CTX-1)**
NEUE AMINOSÄURESEQUENZEN MIT MIKROBIZIDER WIRKUNG, AUS CARDIOTOXIN 1 VON NAJA ATRA ENTSTAMMEND
NOUVELLES SÉQUENCES D'ACIDES AMINÉS AVEC ACTIVITÉ ANTIMICROBIELLE, DÉRIVÉS DE LA CARDIOTOXIN 1 DE NAJA ATRA

(30) Priority: 19.05.2015 IT UB20150719
(43) Date of publication of application: 28.03.2018
(73) Proprietor: Università degli Studi di Parma, 43121 Parma (IT)
(72) Inventor: CABASSI, Clotilde Silvia, 43126 Parma (IT); SALA, Andrea, 43126 Parma (IT)
(74) Representative: Botti & Ferrari S.p.A.
(86) International application number: PCT/EP2016/061022
(87) International publication number: WO 2016/184855

(56) References cited:
- WO-A1-93/05153
- WO-A2-2014/102596

## Description

### FIELD OF INVENTION

The present invention provides a series of cationic peptides with antimicrobial, cytostatic or cytotoxic activity towards at least one Gram negative and/or Gram-positive bacterium and/or a fungus (including yeasts) and/or virus, derived from reference strains or clinical isolates.

### STATE OF THE ART

Antibiotic-resistance, defined as the emergency (and propagation) of bacterial factors of resistance against antibiotics, is mainly triggered by selective pressure, exerted on microbial populations through the use of these drugs. In human medicine, the antibiotic resistance is reaching worrying proportions by both frequency and speed of dissemination, creating serious problems of therapy and endangering affected patients survival. Infections sustained by resistant bacteria lead to an health costs estimated for about 4 billion $ per year in the US alone, with increased morbidity, mortality and disease-associated costs. This problem has become today a real public health priority worldwide, not only for important clinical implications (increased morbidity, lethality, duration of the disease, possible development of complications, risk of epidemics), but also for the economic impact of antibiotic-resistant bacteria, due to increased costs required for the use of more expensive drugs and procedures, employed because of lengthening of hospital stays, and for any disabling outcomes.

Moreover, the emergence of pathogens simultaneously resistant to most antibiotics (defined as *multidrug resistance,* or *heterogeneous resistance)* further reduces the possibility of an effective treatment. It should be noted that this phenomenon often involves health facilities. These multi-resistant organisms resulting from nosocomial infections can be transmitted in the community in various ways including: ventilation systems, water flows, tissues and laboratory samples handling, staff and environment hygiene deficiency, surgical and invasive aids practices *(*Eggimann, Clin Microbiol Infect 2001; 7: 91*;* Pittet, the Lancet 2000; 356: 1307*;* Hugonnet, Clin Microbiol Infect 2000; 6: 350*;* Pittet, Swiss-NOSO 2001; 8:25*).*

Living organisms defend themselves against the invasion of foreign agents through two types of responses: the so-called "innate or natural" immunity and the "acquired or specific" immunity. Innate immunity is a pre-existing defense mechanism before encountering the antigen. It recurs to the use of several mechanical and chemical factors (skin, saliva, gastric secretion), humoral factors (lysozyme, complement, interferon), cells (phagocytes) and commensal bacteria. Antimicrobial peptides represent a further way of innate immunity against microbial infections. They have double importance as they protect about 80% of animal species and almost all the plants, and they play a key role in higher animals immunity, providing a sort of first defense line that stimulates and actively cooperates with adaptive immune responses *(Tossi and Sandri, 2002).* In higher animals, they represents "effector" molecules of innate immunity *(Boman, 2003).* The identification since the early 80' of Cecropins, Magainins and Defensins in insects, amphibians and humans respectively, has stimulated the research on AMPs and brought to an exponential isolation and characterization of hundreds of new peptides. To date, more than 2000 different AMPs sequences have been recorded. The largest databases of AMPs can be generally classified into three categories: (i) generic databases for natural AMPs (APD, CAMP ANTIMIC, AMSDb), (ii) databases specialized in natural peptide (Peptaibol, PenBase, Cybase, PhytAMP), (iii ) database specialized in artificial AMPs (SAPD, RAPD). All mentioned database were described in scientific papers published between 2002 and 2010.

All AMPs exhibit a broad spectrum of action: they are capable of rapidly killing bacterial cells and appear to be active against many antibiotic-resistant strains of human and veterinary clinical significance *(Hancock and Chapple* 1999; *Zasloff 2002).* Most antimicrobial peptides acts by altering the membrane of target cells *(Thevissen et al. 2000).* Bacterial membranes, unlike those eukaryotic, are rich in anionic phospholipids, such as phosphatidylserine and phosphatidylglycerol, which promotes the electrostatic interaction of the positively charged peptide with the membrane, determining the structural perturbation of the lipid bilayer. Eukaryotic membranes present a high content of zwitterionic phospholipids, such as phosphatidylcholine, phosphatidylethanolamine and sphingomyelin. Furthermore, the presence of high quotes of cholesterol, absent in bacterial membranes, it appears to inhibit the activity of these peptides thus conferring a certain selectivity of action (*Zasloff 2002).*

Another factor that contributes to the selectivity of these molecules, appears to be the membrane potential: the most negative potential of the bacterial cells (100-150 mV) eases the interaction with the lipid bilayer *(Bechinger 1997*). Experimental observations in membrane models have suggested two main mechanisms of action: the "carpet model" and the "pore model".

In the former, antimicrobial activity is expressed by "detergent-like" effect in which the amphipathic structure of peptides, interacting with the lipid bilayer, allows to destroy its own organization, with consequent leakage of cytoplasmic components. In the latter, antimicrobial activity is achieved by the formation of channels due the aggregation of peptide monomers in the double-membrane lipid layer thickness *(The Guernevé et al. 1998*). This mechanism was first described in Shai-Matsuzaky-Huang model (*Zasloff*)*.*

Most of these peptides show one or more disadvantages that limit a potential therapeutic use *(Stein and Raoult 2002).*

For example, the AMP polymyxin B causes nephrotoxicity, neurotoxicity and hyperthermia when used in therapeutically effective concentrations *(Ostronoff 2006).*

Another example is provided by temporin-L, AMP isolated from *Rana temporaria* frog and toxic to eukaryotic cells including those human *(Rinaldi 2002).*

A further example is provided by clavaspirin, an histidine rich antimicrobial peptide, isolated from the tunicate *Styela clava* pharyngeal tissue, and found to be powerfully hemolytic to both human and bovine erythrocytes *(Lee et al. 2001).*

The synthetic peptide KFFKFFKFFK, consisting of cationic lysine residues and hydrophobic phenylalanine residues, was found to be severely hemolytic at 40 µg / ml *(Vaara M. and M. Porro, 1996).*

In a similar manner also bombinin H6, isolated from the *Bombina orientalis* skin secretions, caused marked hemolysis (60%) at a concentration of 4 µM.

It is therefore necessary to identify those antimicrobial peptides that have a high biological activity against microorganisms, associated with a low or no toxicity toward eukaryotic cells, and characterized by a broad spectrum of activity, high structural stability and resistance to proteases for different clinical applications.

There is a class of peptidic toxins (called crotamins), first discovered within the venom produced by snakes belonging to the species *Crotalus durissus terrificus,* which, as well as presenting a similar structure to that of the defensins, are considered to be cell-penetrating. Recently it was shown that they are also expressed on the mucosal and epithelial surface, displaying an antimicrobial activity that strengthens their role as real AMPs.

Different *phyla* belonging to the animal kingdom include many venomous species; among these, can be cited coelenterates, flatworms, annelids, echinoderms, mollusks, arthropods and chordates *(B.G. Fry et al 2009).* These animals are able to produce a wide variety of toxins used for prey immobilization and defense against predators. Chemical analyzes have shown that these toxins are generally proteins.

Some proteic components of venoms, secreted by some species of medical importance (such as spiders, scorpions, snakes, and fish) are still extensively studied for their antipain properties *(SP Park et al, 2008; Gomes A. et al 2014)* or anticancer effects(DeShane *J. et al 2003; Abd El-Dayem SM et.al 2012).*

Within the reptiles class there are many species that have evolved venom production and inoculation systems. *Squamata* order includes lizards and snakes, and houses within itself the totality of venom-producing species of reptiles. Of the approximately 3,000 species of snakes identified to date, five large families are venomonous. These families are: *Elapidae* (cobras, mambas, coral snakes and sea snakes), *Viperidae* (vipers and rattlesnakes), *Atractaspididae* (stiletto snakes) and, in a limited way, *Colubridae.* Elapid three-finger toxins constitute a large superfamily of non-enzymatic polypeptides.

The great variety of sequences and their different functional activities, provide a field of interest for many biochemical and biomedical researchers. The members of this family contain 50 to 74 amino acid residues and multiple disulfide bridges, with 4 of them being conserved in all family members. Due to this they share a similar folding pattern which consists in a structure composed of 3 loops emerging from a globular core, stabilized by the 4 invariant disulfide bridges.

Cardiotoxins (CTXs) are the most important group of these polypeptides consisting of about 60 amino acids and abundantly present in the venom of snakes belonging to the Elapid family. All CTXs present β sheet structures that adopt the typical "three finger fold" topology. The CTXs affinity for the lipid bilayer mainly depends on the amino acid sequence of the three loops apex.

Within the large superfamily of three finger toxins, composed of 33 different groups, cytotoxins belonging to Type IA (which includes the cardiotoxins) represent an important subgroup and are produced mostly by Asian snakes belonging to genus Naja (cobra). The three-dimensional structure of these toxins reveals an asymmetrical distribution of the hydrophobic and hydrophilic amino acids. Two distinct types of CTXS, called P-type and S-type, have been characterized. P-type toxins are characterized by the presence of proline 31 within a region called "phospholipid binding site" near to the apex of the loop II. S-type toxins are instead characterized by the presence of serine 29 within the same, but more hydrophilic, region. P-type configuration exhibits increased merger and binding activity than S-type, probably due the additional phospholipid binding site on the loop II. Phospholipid binding site regions show a wide variation in their amino acid residues while maintaining an almost common distribution of the types of residues. The two phospholipid binding sites are positioned between residues 6-13 and 24 - 37, next to the loop I and II apex, respectively.

Within this group, Chen *et al* observed an antimicrobial activity exerted by the cardiotoxin 3 (CTX-3), produced by *Naja atra,* against *Staphylococcus aureus* and *Escherichia coli.* This activity is related with an increased permeability of the bacterial membrane, following to its loss of integrity. Furthermore, it was also observed cardiotoxin 3 binding ability to the lipopolysaccharide (LPS) and lipoteichoic acid acid (LTA) . WO-A2-2014102596 (Università di Parma) discloses antimicrobial peptides from cardiotoxins, though with quite different structures.

To date antibacterial and antimicrobial activity of other known cardiotoxins is unknown.

Many have attempted to identify the region responsible for the antimicrobial activity inside CTX-3 sequence, and the "core peptide", which is the shorter sequence showing a significant antimicrobial activity. This "core peptide" may be used as template for the development of new antibiotics. Literature data suggest that loops I and II apexes interact with membranes, indicating that "core peptide" may coincide with these sequence fragments.

### SUMMARY

Object of the present invention is a series of linear peptides with broad spectrum of action, high antimicrobial, antiviral and antifungal activity, together with further useful features of non-toxicity and permanence of activity in unfavorable environmental conditions.

The peptides of the invention have a length of 20 amino acids and the following structure:

A-B-C-D-E-F-G,

in which:
- A represents a basic amino acid,
- B, D, F represent respectively 5, 3 and 3 amino acids, chosen from the group of hydrophobic amino acids,
- C, E represent 3 amino acids, where each C and E comprises at least one basic amino acid and at least one hydrogen bonds- forming amino acid,
- G represents 2 amino acids, chosen from basic amino acids and / or hydrophobic amino acids, their salts and / or mixtures thereof.

The invention also identifies favourite subfamilies of such peptides and specific peptidic sequences in conformity with the above structure. Unexpectedly, the peptides in accordance with the invention have shown a synergistic combination of interesting characteristics from the point of view of antibacterial applications, in particular: a high level of cell membrane penetration capability, high activity against Gram-positive microorganisms, high activity against Gram-negative microorganisms, high activity against yeast, and high salt-resistance (permanence of antibacterial activity in presence of increasing concentrations of NaCl). These compounds also results extremely tolerable to the body, thus allowing the development of an effective antibacterial therapy with a high therapeutic index. The invention includes a process for the synthesis of these peptides, their pharmaceutical compositions, and their use in the treatment of bacterial and fungal diseases.

### DESCRIPTION OF FIGURES

Figure 1 - Representation of the peptide NCP-2: A -structure; B - surficial positive charge distribution; C - distribution of charged amino acids (dark gray) and hydrophobic amino acids (light gray); D- hydrophobic and hydrophilic portions distribution of peptide NCP-2
Figure 2 - Representation of the peptide NCP-3: A -structure; B - surficial positive charge distribution; C - distribution of charged amino acids (dark gray) and hydrophobic amino acids (light gray); D- hydrophobic and hydrophilic portions distribution of peptide NCP-3
Figure 3A: permeabilization assay using NCP0 of outer and inner membranes made by colorimetric evaluation with CENTA and ONPG, respectively. ◆: damage to the outer membrane; □: damage to the inner membrane.
Figure 3B: permeabilization assay using NCP2 of the outer and inner membranes made by colorimetric evaluation with CENTA and ONPG, respectively. ◆: damage to the outer membrane; □: damage to the inner membrane.
Figure 3C: permeabilization assay using NCP3 of the outer and inner membranes made by colorimetric evaluation with CENTA and ONPG, respectively. ◆: damage to the outer membrane; □: damage to the inner membrane.
Figure 4: Hemolytic activities of NCP-2 and NCP-3.

### DETAILED DESCRIPTION

In the present text the term "treatment" means the effects of the invention peptides capable of imparting a benefit to patients suffering from an infectious disease, such as an improvement in the patient's condition or a delay in disease progression.

"Infection" or "infectious disease" mean the invasion, colonization and / or the multiplication of a microorganism in or on another host organism.

A "microbial infection" is an infectious disease caused by a pathogen.

"Subject" or "patient" is any multicellular organism, including a human, an animal, plant or insect that can be infected with a microorganism. Preferably, the subject is a human or animal organism.

"Peptide" is defined by a multiplicity of amino acid residues joined by peptide bonds; it is the same as "polypeptide" and "protein" and can be used interchangeably.

A "series", in the present document, is defined as any possible variants of the peptide of the invention, wherein one or more amino acids of the peptide sequence are substituted with a homologous amino acid so that the properties of the peptides are retained, even if not necessarily at the same level. A variant can show a higher or lower activity and/ or a wider spectrum (for example, activity against a wider range of microbes), or be more specific for a particular microorganism. Preferably, conservative substitutions of amino acids are carried out in one or more amino acid residues.

A peptide with "alpha helix" structure is a peptide in which the sequence of links Cα-C-N-Cα of the amino acid residues is wrapped around an imaginary central axis. The -R side groups of the amino acid residues protrude radially outwardly of the helix. A complete revolution of the helix involves 3,6 amino acids and corresponds to a distance of 5,4 Å along the imaginary axis.

All amino acids present in this peptide can be either in the D- or L-form; preferably, they are predominantly (i.e. at least 50%) or totally in the L- form. Another preferred aspect relates to the peptides consisting of at least 80%, for example 90%, or more preferably 91-100%, for example 100%, of L-amino acids. Another preferred aspect of the invention relates to peptides constituted of at least 80%, for example 90%, or more preferably 91- 100%, for example 100%, of D-amino acids.

In accordance with the invention, hydrophobic amino acids are selected from: alanine (A), phenylalanine (F), isoleucine (I), leucine (L), proline (P), tyrosine (Y), tryptophan (W) and valine (V).

Basic amino acids of group are selected from: lysine (K), histidine (H), arginine (R).

Amino acids forming hydrogen bonds are selected from asparagine (N), glutamine (Q), serine (S), threonine (T).

The groups C and E, in addition to amino acids, basic and forming hydrogen bonds, mandatory) can optionally contain one or more of: glycine, leucine, isoleucine.

In said structure A-B-C-D-E-F-G, A is the NH2-terminal residue and G is the COOH-terminal one.

Residues B, C, D, E, F and G contain more than one amino acid, as described in the summary. Within each residue (provided that the type of amino acid composition that characterizes it, as indicated in the summary is respected), amino acids can be equal and / or different to each other. For example, in the case of B hydrophobic residue, it may indifferently represent sequences as: AFPVY, AAFPY, AFIWA, LIAAA, FAYAA, APAAA, AAAAA, etc., where the repeated amino acid is highlighted (A in this case).

The invention also provides the following preferred definition for C, A, G and E residues:
- C is preferably represented by the amino acid sequence SKT (serine-lysine-threonine)
- A is preferably represented by the amino acid K (lysine)
- G is preferably represented by the amino acid sequence KI (lysine-isoleucine)
- E is preferably represented by the amino acid sequence IKN (isoleucine-lysine-asparagine) of GKN (glycine-lysine-asparagine).

Other preferred definitions for B, D and F residues are:
- B is preferably represented by the amino acid sequence LIPIL (leucine-isoleucine-proline-isoleucine-leucine), LIWIL (leucine-isoleucine-tryptophan-isoleucine-leucine), LIPIA (leucine-isoleucine-proline-isoleucine-alanine), LIFIL (leucine-isoleucine-phenylalanine-isoleucine-leucine), LILIL (leucine-isoleucine-leucine-isoleucine-leucine), LIYIL (leucine-isoleucine-tyrosine-isoleucine-leucine), or any other hydrophobic amino acid combinations including W (tryptophan), preferably tryptophan in position 4, numbering the sequence from A to G
- D is preferably represented by the amino acid sequence IPA (isoleucine-proline-alanine)
- F is preferably represented by the amino acid sequence LFY (leucine-phenylalanine-tyrosine), LIY (leucine-isoleucine-tyrosine), LLY (leucine-leucine-tyrosine), or LVY (leucine-valine-tyrosine).

In the present invention the peptide variants containing W (tryptophan) are shown particularly suitable to penetrate within the membranes and spread therein. Particularly the tryptophan residues exhibit the peculiar property of interacting with the interfacial regions therefore firmly anchoring the peptide to the surface of the bilayer. This ability is attributable in particular to the aromatic side chain capable of forming hydrogen bonds also due to its dipole moment of about 2.1 D (Bi X. et al 2013).

In the peptide of the invention, one or more of the preferred definitions described for A, B, C, D, E, F and G may be present, independently of one another.

According to more preferred variants, in the structure A-B-C-D-E-F-G, the C residue represents SKT. Also further preferred are the structures in which:
- one or more of the residues A, G, E is represented by one preferred definition thereof as indicated above, and / or
- one or more of the residues B, D, F is represented by one preferred definition thereof as indicated above.

In a particularly favoured variant in the above mentioned structure A-B-C-D-E-F-G: A is K, C is SKT, D is IPA and G is KI.

Finally, the invention comprises a series of specific sequences in accordance with the structure A-B-C-D-E-F-G, where each residue has a defined composition. These sequences are the following, corresponding to the following SEQ.ID.NO. and to the following internal references used in the present description:

| A | B | C | D | E | F | G | SEQ.ID.NO. | Code |
|---|---|---|---|---|---|---|---|---|
| | | | | | | | | |
| K | LIPIL, | SKT | IPA | IKN | LFY | KI | 01 | NCP2 |
| K | LIWIL | SKT | IPA | IKN | LFY | KI | 02 | NCP3 |
| K | LIPIA | SKT | IPA | GKN | LFY | KI | 03 | NCP1A |
| K | LIPIA | SKT | IPA | GKN | LIY | KI | 04 | NCP1B |
| K | LIPIA | SKT | IPA | GKN | LLY | KI | 05 | NCP1C |
| K | LIPIA | SKT | IPA | GKN | LVY | KI | 06 | NCP1D |
| K | LIFIL | SKT | IPA | IKN | LFY | KI | 07 | NCP3A |
| K | LILIL | SKT | IPA | IKN | LFY | KI | 08 | NCP3B |
| K | LIYIL | SKT | IPA | IKN | LFY | KI | 09 | NCP3C |
| | | | | | | | | |

The structure A-B-C-D-E-F-G defined, presents particular chemical and physical features. Therein are distinguished, on a 20-amino acids specific length: 3 hydrophobic regions (residues B, D, F), one of which (B) being particularly broad. This quantity and disposition of hydrophobic amino acids is such as to permeate and possibly lyse the bacterial membrane, however without disrupt the membranes of eukaryotic cells, providing a poor / no toxicity to the patient. The present invention has the characteristic that residues B, D and F exclusively contain hydrophobic amino acids, constituting extended hydrophobic areas and highly homogeneous from this point of view. The above-mentioned hydrophobic zones are alternated with charged areas (residues C and E) being inherently inhomogeneous: in fact, both the C and the E residue contain both basic and hydrogen bonds-forming amino acids. The above-mentioned A-B-C-D-E-F-G structure, particularly the alternation of hydrophobic amino acid in discrete regions, separated by charged, or basic and / or hydrogen bonds-forming amino acids, gives a high activity to the peptides; the resulting structure is also characterized by a high degree of salt-resistance (maintaining activity in the presence of high concentrations of NaCl), as well as a high solubility in aqueous solvents. Finally the end portions of the sequence of (A and G) partly characteristics that are partly shared (basic amino acids) and partly not shared (hydrophobic amino acids, only in the case of G). The peptide, according to the invention, shows a high tendency to take a spatial α helix conformation. The peptides object of the present invention are easy to be synthetized, highly effective, proteolytically stable, substantially salt-resistant, non-hemolytic and slightly cytotoxic for eukaryotic cells.

Due to their strong antibacterial / antifungal / antiviral activity, the previously described peptides may be used in the treatment of related bacterial and / or fungal and / or viral pathologies. Particularly, they may be employed in the treatment of infections in various districts of the human or animal body, (for example, pulmonary, gastrointestinal, urinary), skin infections and medical or surgical diseases complicated by further bacterial, fungal or viral infections. Therefore, the invention concerns the use of a peptide having the above defined structure A-B-C-D-E-F-G for the manufacture of a medicament useful for the prevention or the treatment of a subject suffering from a bacterial and / or fungal and / or viral infection. The invention also extends to the same peptide for use in the prevention or treatment of a subject suffering from an infection from bacteria and / or fungi and / or viruses. The invention also extends to a method of preventing or treating bacterial / fungal / viral infections, comprising administering a dose of the said peptide to a patient suffering from the said infections. In a first variant, the treatment is particularly directed against Gram negative bacteria; in a second variant, the treatment is directed against Gram-positive bacteria; in a third variant the treatment is directed against fungi; in a fourth variant the treatment, is directed against viruses; in a fifth variant the treatment, is directed against micro-organisms belonging to more than one of the above groups.

In the case of treatments on humans or animals, dosage units for said peptides are generally comprised in the range between 1 and 5 mg. Useful doses for said peptides are generally comprised in the range between 0.5 and 2.5 mg/kg.

Above-mentioned peptides can be effectively used as primary agents or adjuvants in the treatment of infectious diseases of both human and animal different districts of the organism. In addition, the peptides of the invention may also be employed in the treatment of infections of plant organisms. Collectively, or individually, the peptides of the present invention may also be useful in the treatment of associated infections, generally of the skin including, and not limited to, ulcers and lesions, skin wounds, cuts or burns. A further preferred aspect of the invention indicates that the peptides, collectively or individually, are useful in the treatment of bacterial skin infections or pyoderma. Another aspect involves the use of the peptides of the invention, singly or in mixtures, in the treatment of diseases (clinical or surgical) complicated by further bacterial infections including, but not limited to, mucosal associated infections, gastrointestinal tract associated infections, urogenital infections, infections of the urinary tract (for example, pyelonephritis, cystitis, urethritis) or respiratory infections, for example, cystic fibrosis.

A particularly advantageous aspect of the invention is represented by the salt-resistance of the peptides herein described. This characteristic allows them to retain a significant / high antimicrobial activity even in particular pathological areas subject to infections, which involve high concentrations of NaCl, such as lung fluid in cystic fibrosis.

The bacterium object of the present treatments can be selected for example in the group including: Staphylococcus spp., in example Staphylococcus aureus (e.g. Staphylococcus aureus ATCC 25923), Enterococcus spp., for example Enterococcus faecalis ATCC 29212; Pseudomonas spp., for example Pseudomonas aeruginosa ATCC 27853; Mycobacterium spp., for example Mycobacterium tubercolosis; Enterobacter spp.; Campylobacter spp.; Salmonella spp. (e.g. Salmonella enteriditis ATCC 13076); Streptococcus spp., for example Streptococcus group A o B, Streptococcus pneumoniae, Helicobacter spp., for example Helicobacter pylori; Neisseria spp., for example Neisseria gonorreae, Neisseria meningitis; Borrelia burgdoferi, Shigella spp., for example Shigella flexneri; Escherichia coli (ATCC 25922); Haemophilus spp., for example Haemophilus influenzae; Francisella tularensis, Bacillus spp., for example Bacillus anthracis; Clostridium spp., for example Clostridium botulinum, Yersinia spp., for example Yersinia pestis; Treponema spp.; Burkholderia spp., for example Burkholderia cepacia ATCC 17759, B. mallei e B. pseudomallei; Stenotrophomonas spp., for example Stenotrophomonas maltophilia ATCC 13637; for example Acinetobacter baumanii.

A fungal pathogen may be derived from one of the fungi (including yeasts) belonging to the group including the genera: Candida spp. (e.g. C. albicans), Epidermophyton spp., Exophiala spp., Microsporum spp., Trichopyhton spp. (for example T. rubrum and T. interdigitale), Tinea spp., Aspergillus spp., Blastomyces spp., Blastoschizomyces spp., Coccidioides spp., Cryptococcus spp. (for example C. neoformans), Histoplasma spp., Paracoccidiomyces spp., Sporotrix spp., Absidia spp., Cladophialophora spp., Fonsecaea spp., Phialophora spp., Lacazia spp., Arthrographis spp., Acremonium spp., Actinomadura spp., Apophisomyces spp., Emmonsia spp., Basidiobolus spp., Beauveria spp., Chrysosporium spp., Conidiobolus spp., Cunninghamella spp., Fusarium spp., Geotrichum spp., Graphium spp., Leptosphaeria spp., Malassetia spp. (e.g. Malassetia furfur), Mucor spp., Neotestudina spp., Nocardia spp., Nocardiopsis spp., Paecilomyces spp., Phoma spp., Piedraia spp., Pneumocystis spp., Pseudallescheria spp., Pyrenochaetas spp., Rhizomucor spp., Rizhopus spp., Rhodotorula spp., Saccharomyces spp., Scedosporium spp., Scopulariopsis spp., Sporobolomyces spp., Syncephalastrum spp., Trichoderma spp., Trichosporon spp., Ulocladium spp., Ustilago spp., Verticillium spp., Wangiella spp.

In a preferred variant, though not limiting, the bacterium being target of the treatment is chosen in the group of: Pseudomonas spp., Escherichia spp., Acinetobacter spp., Klebsiella spp., Enterobacter spp., Burkholderia spp., Proteus spp., Moraxella spp., Streptococcus spp., Staphylococcus spp., Enterococcus spp., Mycobacterium spp., Candida spp. o Malassetia spp.

The virus being target of the treatments can be chosen, for example, in the group comprising enveloped viruses, for example: Herpesviridae (e.g. HSV-1, CMV, BoHV-1), Orthomyxoviridae (for example the flu A virus), Paramyxoviridae (for example Measles virus and distemper virus of the dog), Rhabdoviridae (for example rabies virus), Arenaviridae (for example Lassa virus), Filoviridae (such as Marburg and Ebola viruses), Togaviridae (such as Chikungunya virus and equine encephalitis virus), Coronaviridae (such as SARS virus and Infectious Bronchitis of Chicken), Retroviridae (such as HIV and feline infectious immunodeficiency virus), Hepadnaviridae (such as hepatitis A, B and C virus).

The antimicrobial activity of a peptide according to the invention can be determined using a well-known method, for example the broth dilution. Essentially, this method involves growth of a microorganism in a liquid medium until the logarithmic phase. The peptide to be tested is serially diluted with the growth medium for the bacterium under investigation in wells of a multiwell plate. Optimal concentration of the microorganism is added into wells containing the serially diluted peptide. The plate is incubated in a thermostat at a temperature of 37°C for a sufficient time for the microorganism growth. The growth of the microorganism, compared with a negative control (micro-organism grown in the absence of the peptide), is determined by detecting the absorbance of the solution containing the bacterium, for example, at 605 nM.

Another method, known in the art, to determine the antimicrobial activity of a peptide of the invention peptide is the agar diffusion test. Essentially, the test is performed on agar plates of 14 cm or 9 cm. To make an inoculum of bacteria on the agar, 4-5 colonies grown on the isolation primary medium are suspended in 4-5 ml of Tryptic Soy Broth (enrichment broth), incubating for 2-6 hours until the broth culture has reached an opacity corresponding to 0.5 of the nephelometric scale McFarland (i.e. 1.5 x 106 bacteria). The bacterium under investigation is spread on the agar surface. Subsequently, disks soaked with the peptide are deposited on the surface of the perfectly dried agar using sterile tweezers and after making them adhere to the surface. The plate is incubated in a thermostat at a temperature of 37°C for 24 hours. The diameter of the inhibition zones will allow to define the bacterium resistant, in intermediate resistance or sensitive to the peptide.

For example, the activity on Gram-negative bacteria has been verified with reference to bacteria such as Pseudomonas aeruginosa, Escherichia coli, Moraxella catarrhalis, Acinteobacter baumanii, Enterobacter cloacae, Klebsiella pneumoniae subsp. pneumoniae e Burkholderia cepacia. Pseudomonas aeruginosa is an especially problematic Gram-negative bacterium because of its invasiveness and heterogeneous resistance to antibacterial chemotherapy. This organism is responsible for severe infections and causes of major morbidity in subjects immunocompromised by viral infections such as HIV, cancer chemotherapy or immunosuppressive therapy. Moreover, this bacteria is often the causative agent of serious infectious diseases of lower respiratory tract, urinary tract, skin lesions (wounds, ulcers) in young people, including those with cystic fibrosis, and elderly hospitalized patients. In recent years, the incidence of Pseudomonas infections, in cystic fibrosis, is dramatically increasing.

Escherichia coli is a Gram-negative microorganism belonging to the Enterobacteriaceae family which includes bacteria such as Shigella, Salmonella, Klebsiella or Proteus. Escherichia coli is an important pathogen of infectious diseases, often causing urinary tract infections, bacteraemia, nosocomial and communities pneumonia and various infectious diseases of the abdominal cavity. The emerging resistance to antibacterial chemotherapy seen in recent years in Escherichia coli is becoming a serious health problem. Particularly interesting is the resistance related to the production of broad-spectrum beta-lactamase, which made this bacterium resistant to cephalosporins and fluoroquinolones, especially ciprofloxacin.

The activity on Gram-positive bacteria has been verified by reference to bacteria such as Staphylococcus aureus and its methicillin-resistant variant.

The activity on mycobacteria has been verified by reference to fast growing mycobacteria such as My. smegmatis and My. fortuitum. In contrast to traditionally mycobacteria cause of disease in animals and humans, who require a growing incubation period of at least 15 days, such mycobacteria defined "fast growing" allow to obtain a quantifiable growth within 24 hours and therefore they represent an excellent candidate for the evaluation of anti-mycobacterial activity. Moreover, the transition into a dormant non-replicative state under hypoxic conditions was shown (Dick T, Lee BH, Murugasu-Oei B, 1998: oxigen depletion induced dormancy in Mycobacterium smegmatis. FEMS Microbiol Letter, 163:159-64) thus Mycobacterium smegmatis is the ideal candidate to test anti-tuberculosis agents in bacteria not replicative phase.

The biological activity of the peptides of the invention against a fungus can be for example determined by microdilution broth assay and colony count in plate. In the present invention the ability was evaluated to inhibit the growth and/or to kill fungi (including yeasts) belonging to the genus Candida spp. or Malassetia spp. such as Candida albicans or Malassetia furfur. Malassetia, previously known as Pityrosporum, is a fungi genus that live on the skin of many animals, including humans, and occasionally cause of opportunistic infection. Recently, using molecular biology techniques, has been observed that this fungus is the pathogenic agent of many humans dermatitis, includes dandruff and seborrheic dermatitis (Sugita T, Tajima M, Takashima M, et al., 2004, "A new yeast, Malassetia yamatoensis, isolated from a patient with seborrheic dermatitis, and its distribution in patients and healthy subjects", Microbiol. Immunol. 48 (8): 579-83). It was also discovered that the skin rash observed in tinea versicolor are due to infection by this fungus (Guillot J, Hadina S, Guého E, 2008, "The genus Malassetia: old facts and new concepts". Parassitologia 50 (1-2): 77-9). Malassetia spp. genus members, particularly Malassetia pachidermatis, are of great importance in veterinary medicine. They are saprophytes on the skin and in the external ear canal of dogs and cats as well as other mammals. It is strictly host-adapted, so that is not found free in nature. It is an opportunistic fungus that becomes pathogenic only in presence of contributing factors such as excessive humidity, increased secretion of ear wax or sebum, skin folds. It constitutes one of the most frequent agents of dogs' external otitis. Topical therapy appears to be indicated in the management of ear infections by Malassetia spp., where there is a need of multi-purpose products with simultaneous fungicide/bactericide activity.

The antiviral biological activity of the peptides of the invention can be determined against viruses without or with a pericapsid or envelope. The latter have a bilaminar structured lipidic external limiting membrane or envelope, with transmembrane proteins. The pericapsid originates from the plasmatic membrane of the host cell, by which the virus covers itself during the budding process put in place to leak from the cell. As non-limiting example, the activity was tested against viruses provided with envelope that can infect animals, particularly to BoHV-1 (Bovine Herpesvirus 1).

The bacterial membrane damage can be evaluated by a method essentially consisting in the contact, in a liquid medium, of the microorganism with a peptide. The liquid medium is added with a molecule capable of crossing the intact membrane of the microorganism. Once entered, it is processed inside the microorganism to form a product which is no longer able to cross the membrane. The medium in which the microorganism is suspended is analysed for the presence of the said product. The presence of such product in the incubation medium is an index of the membrane damage caused by the peptide, and is indicative of peptide anti-microbial effect. An example of a suitable molecule is AM calcein. Inside the organism AM calcein is converted to free calcein. Normally, free calcein is not able to cross the microorganism cell membrane and be present in the culture medium. Thus, the detection of free calcein in the medium is an index of microorganism cell membrane damage, and thus of peptide antimicrobial activity.

Another useful method to identify bacterial membrane damage exploits the ability of β-galactosidase of converting a chromogenic artificial substrate known as ortho-nitrophenyl-β-D-galactopyranoside (ONPG) to o-nitrophenol and glucose. This method, originally described by Jeffrey Miller in 1972 and published on "Experiments in Molecular Genetics" was subsequently modified by Zhang and Bremer ninel 1995 (The Journal of Biological Chemistry, 1995, 270, 11181-11189). Essentially this method consists in mixing in a suitable liquid medium an aliquot of bacterial cells and a permeabilizing agent, for example a detergents such as Triton-X 00 or Tween 20, or the peptides of the invention, which destroy the bacterial membranes leaving intact the β-galactosidase enzyme. After a suitable incubation period, a ONPG solution is added to the culture medium. The released β-galactosidase from damaged bacteria metabolize ONPG releasing free nitrophenol. The nitrophenol, which gives a yellow colouration to the medium, is quantified by spectrometric analysis at 405 nM.

In the present invention the peptides cytotoxicity was also determined, for example, by determining haemolysis of red blood cells and classifying the antimicrobial peptides based on their minimal haemolytic concentration. This document defines MHC10 as the concentration of peptide that determines the 10% of haemolysis, MHC50 as the concentration of peptide that determines 50% of haemolysis and MHC90 as the concentration which causes 90% of haemolysis. Peptides that are in the class defined MHC10 at a concentration of 100 micrograms/ml were selected.

The cytotoxicity of the peptides can be assessed by standard methods, for example against the bovine MDBK cell line. The cells are cultured in the presence and in the absence of peptides, and the membranes damage is evaluated using Trypan Blue vital dye.

Mammals, birds and, in general, other animals may be treated with the peptides described in the present invention. Mammals and birds include, but are not limited to, humans, dogs, cats and pet birds and farm animals, such as horses, cattle, sheep, goats, pigs, chickens and turkeys and poultry.

A preferred aspect of the invention involves the use of peptides in the treatment of infectious diseases: infections by Klebsiella, Salmonella, Yersinia, Proteus, and Colibacillosis of pets and production animals.

A further preferred aspect relates to the treatment of glanders in equines and mieloidosis in carnivores and Pseudomonas aeruginosa infections in affection and production animals.

A further aspect relates to the treatment of infection by Bordetella spp., in affection and production animals; infection by Moraxella spp.; Francisella spp., Brucella spp., Campylobacter spp., Pasteurella spp., Actinobacillus spp. (actinobacillosis), Haemophilus spp., Streptococcus spp. (including cattle mastitis and strangles), Staphylococcus spp. (including mastitis, pyoderma, endometritis), infection by Bacillus spp. (including anthrax), Clostridium spp. (including tetanus, botulism and anthrax symptomatic), Listeria spp. (listeriosis), infections by Erysipelothrix spp. (including erysipelas), Mycobacterium spp. (including tuberculosis, paratuberculosis), Leptospira spp., Serpulina (superficial necrotic enteritis), Treponema spp. (Rabbit Syphilis), Borrelia spp. In pets and production animals.

The present invention includes a process for synthesis of the above-mentioned peptides having the above defined structure A-B-C-D-E-F-G. Collectively, or individually, the peptides of the invention are generally synthetic peptides, synthesized in vitro using known chemical methods. For example, they are prepared using the synthesis procedures in solid phase, liquid phase, peptide-condensing procedure, or any combination of these techniques. The amino acids making up the peptides of the invention may be natural or synthetic. Amino acids used for peptide synthesis may be amino acids in which the acid-labile group N-α-t-butyloxycarbonyl (Boc) in accordance with Merrifield work (J. Am. Chem. Soc., 85: 2149-2154, 1963) or the base-labile 9-fluorenylmethoxycarbonyl (Fmoc) as described by Carpino and Han (J. Org. Chem., 37:3403-3409, 1972) protects the α-amino-terminal. Both Boc- or Fmoc- protected amino acids can be obtained from various commercial sources, such as Fluka, Sigma-Aldrich Bachem, Advanced Chemtech, Cambridge Biochemical Research. In general, the chemical synthesis methods on solid phase consist, according to M. Bodansky, Principles of Peptide Synthesis, (SpringerVerlag, Berlin 1984) or JM Stewart and JD Young, Solid-Phase Peptide Synthesis (Pierce Chemical Co., Rockford, Illinois 1984), in the sequential addition of one or more amino acids to the growing peptide chain. Generally, an optimal protecting group protects the amino or carboxylic group of the first amino acid. The first protected amino acid is attached to an inert solid support, for example a resin. The protective group is then removed from the residue bound to the resin and the next amino acids (suitably protected) are added in a sequential manner. After reaching the number of amino acids, all the remaining protecting groups (and any usual support) are sequentially or concurrently removed, to obtain the final peptide.

It is possible to add more than one amino acid at one time to the growing chain, for example, by coupling (under suitable experimental conditions which avoid the formation of racemes, due to the presence of chiral centers) a protected tripeptide with a properly protected dipeptide to form, after deprotection, a pentapeptide as described, for example, by Merrifield in G. Barany and RB Merrifield, The peptides: Analysis, Synthesis, Biology, editors E. Gross and J. Meienhofer, vol.2 Academic Press, New York, 1980, pp 3-254).

The peptide compound purity degree can be determined using various methods, including the identification of the HPLC peaks. Preferably, a peptide that produces a single peak with height and amplitude equal to at least 75% of the input material on an HPLC column is preferred. Even more preferable is a peptide that produces a single peak that is at least 87%, at least 90%, at least 99% or even 99.5% of the input material on an HPLC column.

To ensure that the obtained peptide, using one of the synthesis techniques indicated above, is the desired peptide for the uses or formulations described below in the present invention, the analysis of the peptide composition is carried out with the aid of different analytical methods known in the art. The analysis of the composition can be effected, for example, using the high resolution mass spectrometry to determine the peptide molecular weight. Alternatively, a peptide amino acids content can be confirmed by hydrolysing the peptide in an acidic solution to identify and quantify the components of the mixture using HPLC, or an amino acid analyser. The thin-layer chromatographic methods are also useful: they can also be used to identify one or more constituent groups or residues of a desired peptide.

Another preferred but not limiting aspect of the invention concerns the proportion of water solubility of peptides, between 40% and 90%, preferably between 91% and 97%, even more preferably between 97.5% and 100%, for example 98%. The estimated solubility percentage is calculated by the solubility of two-parameter model of Wilkinson-Harris as described in Wilkinson DL and Harrison RG (1991) Bio/Technology 9, 443-448.

The invention peptides mass can be determined with known procedures, for example, mass spectroscopy. Essentially, this technique involves the ionization of a compound and the subsequent separation of the product ions according to their mass/charge ratio. The mass spectrophotometers have different methods to determine the mass/charge of the ion, for example the flight time. Essentially, the ions originating from the source are accelerated by a potential (V) to the same kinetic energy, then they are left to drift along a tube towards a detector. If a spectrometer possesses a flight tube of length L, the flight time for a given ion is given by the equation: t = (L2*m/2*z*e*V). Starting from the ratio m/z function of the residence time in the flight tube, it can be calculated the mass of a given ion. The flight tube, however, has a low resolution (low ability to discriminate two ions with similar m/z). To overcome this, most of these analysers is equipped with a "reflectron" that is a mirror that reflects the ions making them travel twice the path. In this way, it is possible to distinguish two ions having very similar traveling time in the flight tube. The use of "Reflectron" limits the molecular mass range analysable that is between 200 and 5000-10000 Da.

The secondary structure of peptides in solution can be analysed using a well-known technique referred to as circular dichroism. It is part of chirooptical spectroscopy, meaning those spectroscopic techniques, using polarized light, highlighting the optical activity of the examined molecules. The results obtained with this technique give information on secondary structures percentages present in the polypeptides. Although it is not possible to determine the sequence position, this technique can be used for an initial screening for the choice of the solvent system for NMR analysis and as control of the obtained results from the computation.

Several pharmaceutical formulations containing the invention peptides either singly or combined, can be prepared by procedures described in the art and using known and readily available ingredients. Such formulations constitute part of this invention.

In this document, "excipient" means a compound or its optimal mixture for use in a formulation set up for the treatment of a specific infectious disease or conditions associated with it. For example, an excipient for use in a pharmaceutical formulation should generally not cause an adverse response in a subject. The excipient, as above described, should not significantly inhibit the relevant biological activity of the active compound. For example, an excipient does not inhibit the antimicrobial activity of an antimicrobial peptide of the present invention or a variant thereof. An excipient may simply provide a buffering activity to keep the active compound at a suitable pH to exert its biological activity, for example, saline phosphate buffered. Alternatively or in addition, the excipient can comprise a compound, such as a protease inhibitor, which increases the peptide activity or half-life. In another example, the excipient may include or be itself an additional antimicrobial molecule and/or an anti-inflammatory molecule.

Singly or combined, the peptides of the invention may also be formulated as solutions for oral or parenteral administration, such as intramuscular, subcutaneous, intraperitoneal or intravenous.

The pharmaceutical formulations of the peptides of the invention can take the form of an aqueous solution, an anhydrous form or a dispersion, or alternatively an emulsion, a suspension, an ointment, a cream or an ointment.

Singly or combined, the peptides of the present invention may be included in suspensions, solutions or emulsions in oily vehicles or in water and may contain useful agents for suspending, stabilizing and/or agents favouring the dispersion.

Singly or combined, these peptides can be formulated in powder form, obtained by aseptic isolation of sterile solid or by lyophilisation of a solution, to be reconstituted in solution form with the aid of a suitable vehicle before use, for example injections water.

Singly or combined, the peptides of the present invention can be administered to the respiratory tract. For administration by inhalation or insufflation, the composition may take the form of a dry powder, as an example, a suitable powdered mix as lactose or starch.

Singly or combined, the peptides of the present invention can be administered in an aqueous solution in the form of aerosol or be inhaled.

Throughout the description and the claims indicated below, the words "comprise" and "contain" and variations such as "comprises" and "contains", mean "including but not limited to", and are not intended to exclude other elements. Throughout the description and in the claims indicated below, the singular includes the plural, unless the context requires otherwise. Furthermore, in the parts where the indefinite article is used, the specification indicates both the plurality and the singularity, unless the context requires otherwise.

The present invention is further described in the following, non-limiting, examples.

### EXPERIMENTAL PART

### Example 1: peptide synthesis

Methods: Synthesis of the peptides was performed by the company SelleckChem, through its associate in the United States. The synthesis was carried out by known techniques and specifically by solid phase synthesis.

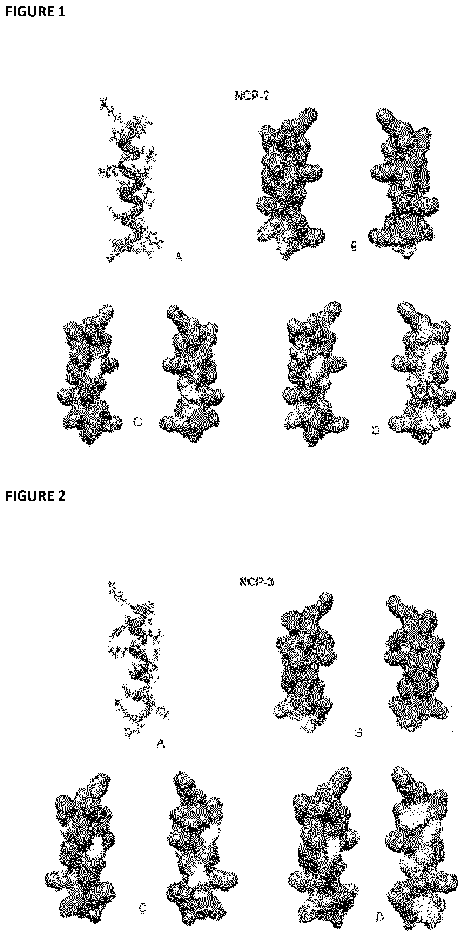

Results: 10 peptides were synthesized, having length of 20 amino acids and with a purity degree greater than or equal to 90%. Peptides were delivered in criovials with 5 mg of lyophilized peptide. The technical reports given by SelleckChem society with peptides have not shown synthesis problems. In Fig.1 is shown the peptide structure, referring to the SEQ.ID.NO.: 01-09, according to the invention. In this text, the SEQ.ID.NO.: 01 is also identified as NCP2, and the SEQ.ID.NO.: 02 as NCP3. Another reference peptide SEQ.ID.NO.:10 was also synthesized and tested , for comparison purpose; this peptide is identified in the text as NCP0 and it does not comply with the structure A-B-C-D-E-F-G of the present invention. The SEQ.ID.NO.:10 (K LIPIA SKT CPA GKN LCY KI) is a synthetic analogue of the amino acid sequence which is the core peptide of Naja atra cardiotoxin; it corresponds to the stretch of protein between first loop apex and second loop base. Despite the similarity with the sequences of the invention, the SEQ.ID.NO.:10 has a partial perturbation at the hydrophobic residues at D and F stretch, which are no longer wholly composed exclusively by hydrophobic amino acid. In this way is altered the alternation sequence between hydrophobic and charged zones, that are characteristics of the invention.

### Example 2: membrane permeabilization assay using E. coli ML-35pYC bacterial strain

Methods: to test the bacterial membrane permeabilization caused by the peptides of the invention, E. coli ML-35pYC strain was used. This bacterial strain express ampicillin resistence, a constitutive expression of cytoplasmic β-galactosidase and is engineered with a plasmid for a periplasmic β-lactamase synthesis. Sixty microliters (60 µl) of bacterial suspension, containing E. coli ML-35pYC strain, were added to 60 µl of ONPG solution (15 mM) or to 60 µl of CENTA (1,5 mM) diluted in phosphate buffer. Subsequently 120 µl of the examined peptide were added, to obtain a final concentration of 12.5 µg/ml (NCP-2=5,4 µM, NCP-3=5.2 µM). The absorbance value was registered at 600 nm for ONPG and at 405 nm for CENTA, every 10 minutes for 240 minutes to assess the reaction's kinetics.

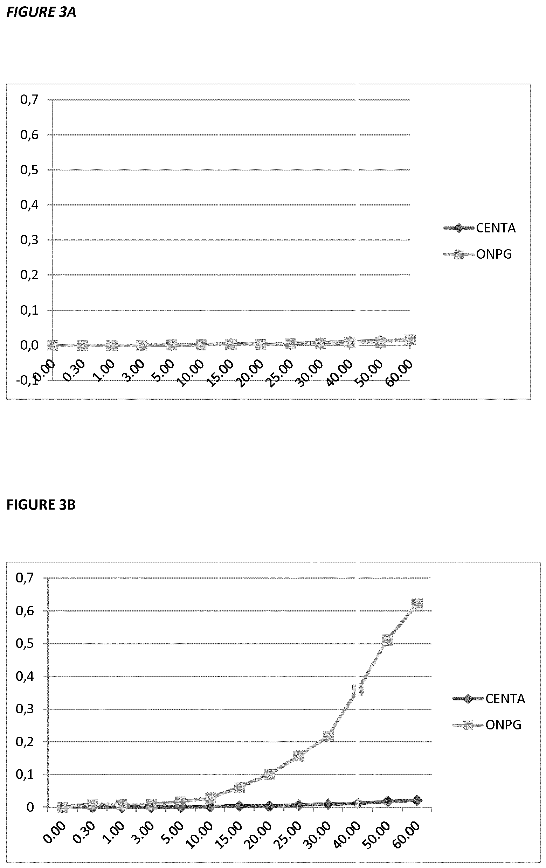

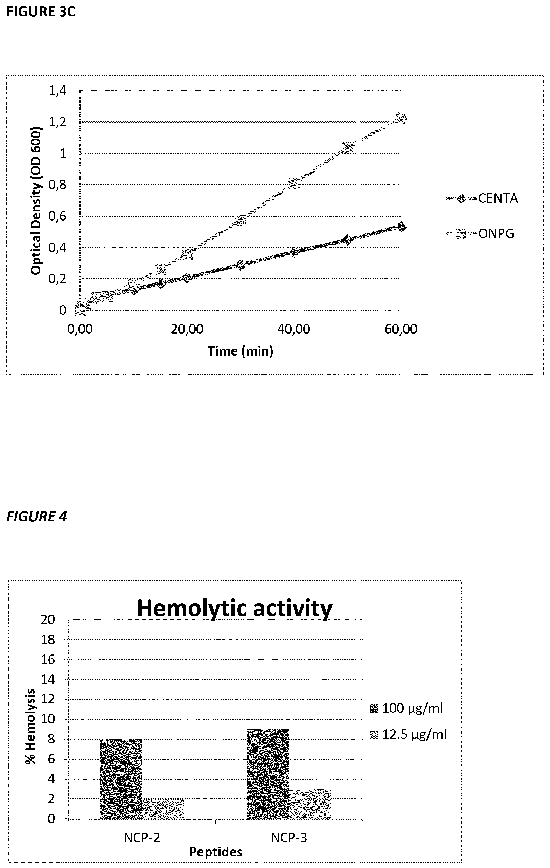

Results: Fig.3A, 3B and 3C showed the permeabilization curves of the 3 tested peptides.

NCP-0 (reference peptide) had not demonstrated activity on outer or inner membrane; this is characterized by the absence of increase of detectable optical density during all the test duration.

NCP-2, according to the invention, showed a penetration of the outer membrane without destruction of the membrane itself; the oscillation of the optical density around the base value during all the test demonstrates this action. The same peptide had a destructive action on the inner membrane, with a linear increase of the optical density.

NCP-3, according to the invention, showed a more intense activity, in particular a lithic action on the inner and outer membranes, with a time-depending increase of the optical density. This action complies with what is reported in literature about antimicrobial peptides.

### Example 3: determination of antimicrobial peptides spectrum of action and efficacy towards Gram-negative bacteria

This example demonstrate the peptides antibacterial spectrum, tested with E. coli ATCC 25922, Pseudomonas aeruginosa ATCC 27853, Proteus mirabilis ATCC 14153, Burkholderia cepacia ATCC 17759, Moraxella catharralis ATCC 25238 and wild strain of Klebsiella pneumoniae subsp. pneumoniae, Enterobacter cloacae, Acinetobacter baumanii. For every peptide and tested strain, LD90 and LD99 (when it is possible) have been determined.

### Methods:

### a) Bacterial inoculum preparation:

For each bacterial strain 3-5 morphologically similar colonies from fresh cultures were taken and inoculated in Brain Heart Infusion broth, then incubated at 37°C in agitation at 225 r.p.m. for about 3-4 hours. Then the bacterial suspension was centrifuged at 1000 G for 20 minutes and the pellet resuspended in phosphate buffer pH 7, 10 mM. The turbidity of the bacterial suspension was measured using a spectrophotometer with absorbance of 600 nm; the range 0.08-0.13 equates to about 10⁸ cfu/ml. The bacterial suspension was diluted 1:100 in phosphate buffer 10mM to obtain a bacterial concentration of about 10⁶ cfu/ml. Within 30 minutes 50 µl of the suspension (10⁶ cfu/ml) were inoculated in each well of the microtiter plate, to obtain a bacterial concentration of about 5x10⁵ cfu/ml.

### b) Serial micro-dilution

The peptides ability to inhibit the bacterial growth in liquid cultures was tested. 50 µl of each peptides solutions of about 0,2-50 µM were added in microtiter plates with 96 wells with U-shaped bottom, containing the liquid culture medium and one of these bacterial strains: E coli ATCC 25922, Pseudomonas. Aeruginosa ATCC 27853, Proteus mirabilis ATCC 14153, Burkholderia cepacia ATCC 17759, Moraxella catharralis ATCC 25238 or other wild strains of Klebsiella pneumoniae subsp. pneumoniae, Enterobacter cloacae, Acinetobacter baumanii.

After 2 hours incubation at 37°C, the bacteria were transplanted on solid medium. As a growth reference, bacterial cultures were grown in absence of peptides.

### c) Transplant on agar:

The bacterial suspension and peptides were put in contact for 2 hours at 37°C, with differing concentrations of the peptide. Then 20 µl from each well of the plate were taken and transferred on solid culture medium, McConkey agar. The inoculum was uniformly distributed on the agar medium with aid of a sterile disposable handle. After an incubation time of 24 hours at 37°C, the bacterial colonies (cfu) were counted.

### Results:

Tab.1 shows LD90 and LD99 values for every peptides. NCP-2 and NCP-3 are particularly active against Pseudomonas aeruginosa ATCC 27853 (LD99 between 3.2 (NCP-2=1. µM and NCP-3=1.3 µM) and 9.67µg/ml). These peptides are also very effective against Acinetobacter baumanii wild strains, with LD99 between 1.6 µg/ml (0.7 µM for NCP-2 and NCP-3) and 55.98 µg/ml (for NCP-2=24.2 µM and NCP-3=23.3 µM). Worthy of note the effect of the 3 peptides against Moraxella catharralis ATCC 25238 (LD99=1.6 µg/ml for each peptides (0.7 µM)). NCP-2 (9.1 µM) action against Proteus mirabilis ATCC 14153 (LD90=2106 µg/ml) is interesting. Conversely the NCP-0 (reference peptide) is scarcely active against almost all Gram-negative tested bacteria.

**Tab. 1: Peptides antimicrobial activity against Gram-negative bacteria. LD90 and LD99 values obtained by micro-dilution tests in liquid medium and subsequent transplant in agar medium**

| **Peptides** | | | | | | |
|---|---|---|---|---|---|---|
| **Gram-negative** | **NCP-0** | | **NCP-2** | | **NCP-3** | |
| | **LD90** | **LD99** | **LD90** | **LD99** | **LD90** | **LD99** |
| *E. coli* ATCC 25922 | 152.02 (70 µM) | ND | 10.445 (4.5 µM) | ND | 7.01 (2.9 µM) | 14.01 (5.8 µM) |
| *Pseudomonas aeruginosa* ATCC 27853 | >100 (46.3 µM) | >200 (92.6 µM) | 3.77 (1.6 µM) | 9.67 (4.2 µM) | 1.76 (0.7 µM) | 3.2 (1.3 µM) |
| *Acinetobacter baumanii* from cloaca (herpetological) | 142.80 (66.1 µM) | 202.98 (94 µM) | 0.8 (0.4 µM) | 1.6 (0.7 µM) | 0.8 (0.3 µM) | 1.6 (0.7 µM) |
| *Acinetobacter baumanii* from skin (herpetological) | ND | 117.96 (54.6 µM) | 0.8 (0.4 µM) | 1.6 (0.7 µM) | 0.8 (0.3 µM) | 1.6 (0.7 µM) |
| *Acinetobacter baumanii* from cloaca (ornithological) | 126.205 (58.4 µM) | 127,26 (59 µM) | 24,46 (10,6 µM) | 55,98 (24,2 µM) | 5,98 (2,5 µM) | 6,4 (2,7 µM) |
| *Acinetobacter baumanii* (human strain) | 123.77 (57.3 µM) | 131.55 (61 µM) | 0.8 (0.4 µM) | 1.6 (0.7 µM) | 0.8 (0.3 µM) | 1.6 (0.7 µM) |
| *Klebsiella pneumoniae* subsp. *pneumoniae* (herpetological) | >100 (>46.3 µM) | >200 (>92.6 µM) | 168.8 (72.9 µM) | 201.1 (86.9 µM) | 37.63 (15.7 µM) | 50 (20.8 µM) |
| *Enterobacter cloacae* (herpetological) | >100 (>46.3 µM) | >200 (>92.6 µM) | 155.9 (67.3 µM) | 221.5 (95.7 µM) | 19.53 (8.1 µM) | 25 (10.4 µM) |
| *Burkholderia cepacia* ATCC 17759 | 39.35 (17.8 µM) | 39.43 (18.3 µM) | 152.0 (65.7 µM) | >152 (>65.7 µM) | 40.25 (16.7 µM) | 50 (20.8 µM) |
| *Proteus mirabilis* ATCC 14153 | >100 (>46.3 µM) | >200 (>92.6 µM) | 21.06 (9.1 µM) | ND | 73.37 (30.5 µM) | 100 (41.6 µM) |
| *Moraxella catharralis* ATCC 25238 | 0.8 (0.4 µM) | 1.6 (0.7 µM) | 0.8 (0.4 µM) | 1.6 (0.7 µM) | 0.8 (0.3 µM) | 1.6 (0.7 µM) |

### Example 4: Determination of peptides antimicrobial spectrum of activity and efficacy against Gram-positive bacteria

NCP-3 show high antimicrobial activity against Gram-positive bacteria (LD99 between 1.6 µg/ml (0.7 µM) and 8.34 µg/ml (3.5 µM) (cf. Table 2) NCP-2 has a great activity against Streptococcus agalactiae ATCC 13813 (LD99=8.73 µg/ml (0.7 µM)); it has LD99 between 50 and 250 µg/ml against MRSA ATCC 43300, Staphylococcus aureus ATCC 25922 and Enterococcus hirae ATCC 10541. The reference peptide (NCP-0) has poor activity against Gram-positive tested strains (LD99>100-200 µg/ml).

### Methods:

### a) Bacterial inoculum preparation:

For each bacterial strain 3-5 morphologically similar colonies from fresh cultures were taken and inoculated in Brain Heart Infusion broth, then incubated at 37°C in agitation at 225 r.p.m. for about 3-4 hours. Then the bacterial suspension was centrifuged at 1000 G for 20 minutes and the pellet resuspended in phosphate buffer pH 7, 10 mM. The turbidity of the bacterial suspension was measured using a spectrophotometer with absorbance of 600 nm; the range 0,08-0,13 equates to about 10⁸ cfu/ml. The bacterial suspension was diluted 1:100 in phosphate buffer 10mM to obtain a bacterial concentration of about 10⁶ cfu/ml. Within 30 minutes 50 µl of the suspension 10⁶ cfu/ml were inoculated in each well of the microtiter plate, to obtain a bacterial concentration of about 5x10⁵ cfu/ml.

### b) Serial micro-dilution:

The peptides ability to inhibit the bacterial growth in liquid cultures was tested. 50 µl of each peptides solutions of about 0.2-50 µM were added in microtiter plates with 96 wells with liquid culture medium and one of these bacterial strains: Staphylococcus aureus ATCC 25923, MRSA ATCC 43300, Streptococcus agalactiae ATCC 13813 and Enterococcus hirae ATCC 10541. After 2 hours of incubation at 37°C, bacterial suspensions were put on solid culture medium. Bacterial cultures were cultivated without peptides as growth control.

### c) Transplant on agar:

The bacterial suspension and differing peptides concentrations were put in contact for about 2 hours at 37°C. After this incubation, 20 µl from each well of the plate were transplanted on solid culture medium, Columbia agar with 5% of bovine erythrocytes for Gram-positive bacteria. The inoculum was evenly distributed on the agar medium with the aid of a sterile disposable handle. After an incubation time of 24 hours at 37°C, the bacterial colonies (cfu) were counted.

### Results:

Tab.2 shows LD90 and LD99 values for every peptides. NCP-3 has a great antimicrobial activity against Gram-positive bacteria (LD99 between 1.6 µg/ml (0.7 µM) and 8.34 µg/ml (3.5 µM)). NCP-2 is mainly active against Streptococcus agalactiae ATCC 13813 (LD99 8.73 µg/ml (8 µM)); it has LD99 between 50 µg/ml (20.8 µM) and 250 µg/ml (108 µM) towards MRSA ATCC 25922 and Enterococcus hirae ATCC 10541. The reference peptide NCP-0 has weak activity against all tested Gram-positive strains (LD99>100-200 µg/(46.3-92.6 µM)).

**Tab. 2: Peptides antimicrobial activity against Gram-negative bacteria. LD90 and LD99 values obtained by micro-dilution tests in liquid medium and subsequent transplant in solid medium**

| **Peptides** | | | | | | |
|---|---|---|---|---|---|---|
| **Gram-positive** | **NCP-0** | | **NCP-2** | | **NCP-3** | |
| | **LD90** | **LD99** | **LD90** | **LD99** | **LD90** | **LD99** |
| MRSA ATCC 43400 | >100 (>46.3 µM) | >200 (>92.6 µM) | >100 (>42.3 µM) | >200 (>84.6 µM) | 6.09 (0.3 µM) | 8.34 (3.5 µM) |
| Staphylococcus aureus ATCC 22953 | >100 (>46.3 µM) | >200 (>92.6 µM) | 36.81 (15.9 µM) | 50 (21.2 µM) | 0.8 (0.3 µM) | 1.6 (0.7 µM) |
| Enterococcus hirae ATCC 10541 | >100 (>46.3 µM) | >200 (>92.6 µM) | 155.09 (65.6 µM) | 240.12 (103.7 µM) | 0.8 (0.3 µM) | 1.6 (0.7 µM) |
| Streptococcus agalactiae ATCC 13813 | 100 (46.3 µM) | >100 (>46.3 µM) | 4.91 (2.1 µM) | 8.73 (3.7 µM) | 0.8 (0.3 µM) | 1.6 (0.7 ) |

### Example 5: Determination of peptides antimicrobial spectrum and efficacy against rapidly growing mycobacteria

NCP-3 showed antimicrobial activity against rapidly growing mycobacteria Mycobacterium fortuitum DSMZ 46621 and Mycobacterium smegmatis DSMZ 43756.

### Methods:

### a) Bacterial inoculum preparation:

Mycobacteria were incubated in Middlebrook 7H9 broth at 37°C for 24 hours. The bacterial suspension was passed through a 25G needle 5-10 times to disrupt cells clusters. Using a spectrophotometer with absorbance of 600 nm it was immediately measured the turbidity of the bacterial suspension; the range 0.08-0.13 equates to about 10⁸ cfu/ml. The bacterial suspension was diluted 1:100 in phosphate buffer 10mM to obtain a bacterial concentration of about 10⁶ cfu/ml. Within 15 minutes 100 µl of the suspension 10⁶ cfu/ml were inoculated in each well of the microtiter plate, to obtain a bacterial concentration of about 5x10⁵ cfu/ml.

### b) Peptide:

The ability of NCP-3 to inhibit bacterial growth in liquid medium was tested. The peptide was diluted in phosphate buffer at concentration of 400 µg/ml (166,4 µM), to obtain a final peptide concentration of 100 µg/ml (41,6 µM). 50 µl of the diluted peptide was put into wells of a microtiter plate; in the wells (final volume: 200 µl) there was EDTA 0,25 nM, 50% Middlebrook 7H9 and one of the following bacteria: Mycobacterium fortuitum DSMZ 46621 and Mycobacterium smegmatis DSMZ 43756 at the concentration indicated above. The microtiter plates were incubated at 37°C for about 72 hours. Bacterial cultures were cultivated without peptide as growth control.

### c) visualization of the bacterial growth:

After 72 hours incubation at 37°C, 20 µl of resazurin 0,01% were added in each well. Reading of results was done after further 24 hours of incubation at 37°C.

Results: NCP-3 at concentration of 100 µg/ml (41.6 µM) inhibited Mycobacterium fortuitum DSMZ 46621 and Mycobacterium smegmatis DSMZ 43756 growth.

### Example 6: peptides antimicrobial activity test against fungal (yeast) strains

### Methods:

Candida albicans ATCC 10231, Candida glabrata ATCC 2001 and Malassetia pachydermatis ATCC 14522 were cultivated in Czapek-Dox broth (DIFCO) medium for 48-72 hours. On the day of the test the fungal culture was centrifuged at 1700G for 15 minutes and the pellet resuspended in phosphate buffer, then put in agitation on an electric vortex to disrupt fungal cell aggregations. The suspension was diluted to get a final optical density of 0,5 of the McFarland scale (about 1-5x106 cell/ml) (Branda J.A., Krantz A. Effect of yeast on automated cell counting. Am J Clin Pathol., 2006:126, 248-254.).

### a) Serial micro-dilution:

Peptides ability to inhibit the fungal growth in liquid cultures was tested. 50 µl of each peptides solutions of about 0.2-50 µM were added in microtiter plates with 96 wells with U-shaped bottom, with liquid culture medium and one of these fungi: Candida albicans ATCC 10231, Candida glabrata ATCC 2001 and Malassetia pachydermatis ATCC 14522. After 2 hours of incubation at 37°C, fungal suspensions were plated on solid culture medium, agar Sabouraud. Fungal cultures were cultivated without peptides as growth control.

### b) transplant on agar:

the fungal suspension and peptides at differing concentrations were in contact for about 2 hours at 37°C. After this incubation, 20 µl from every well of the plate were transplanted on solid culture medium, agar Sabouraud. The inoculum was evenly distributed on the agar with the aid of a sterile disposable handle. After an incubation time of 24 hours at 37°C, the fungal colonies (cfu) were counted.

### Results:

Tab.3 shows LD90 and LD99 values for every peptide. NCP-3 show a good activity towards Candida albicans ATCC 10231, Candida glabrata ATCC 2001 and Malassetia pachydermatis ATCC 14522 (LD99 between 6.4 µg/ml (2.7 µM) and 32.26 µg/ml (13.4 µM)).NCP-2 has an excellent activity against Malassetia pachydermatis ATCC 14522 (LD99 6.3 µg/ml for peptide 2.7 µM); towards Candida albicans ATCC 10231 and C. Glabrata ATCC 2001. NCP-2 has LD99 values of 58.17 µg/ml (peptide at 25.1 µM) and >200 µg/ml (peptide at concentration >68.4 µM) respectively. The reference peptide NCP-0 has a weak activity against all the tested fungal strains (LD99>150 µg/ml for peptide concentration of 69.2 µM).

**Tab. 3: Peptides antimicrobial LD90 and LD99 values (µg/ml) against yeasts, obtained by micro-dilution tests in liquid medium and subsequent transplant in solid medium**

| **Peptides** | | | | | | |
|---|---|---|---|---|---|---|
| **Yeasts** | **NCP-0** | | **NCP-2** | | **NCP-3** | |
| | **LD90** | **LD99** | **LD90** | **LD99** | **LD90** | **LD99** |
| Candida albicans ATCC 10231 | >100 (>46.3 µM) | >200 (>92.6 µM) | 56.91 (25.6 µM) | 58.17 (25.1 µM) | 12.43 5.2 µM) | 13.71 (5.7 µM) |
| Candida glabrata ATCC 2001 | >100 (>46.3 µM) | >200 (>92.6 µM) | >200 (>68.4 µM) | >200 (>68.4 µM) | 30.08 (12.5 µM) | 32.26 (13.4 µM) |
| Malassetia pachydermatis ATCC 14522 | 150.82 (69.8 µM) | 151.87 (70.3 µM) | 2.95 (1.3 µM) | 6.4 (2.8 µM) | 4.156 (1.7 µM) | 6.4 (2.7 µM) |

### Example 7: determination of the spectrum of activity and efficacy of antimicrobial peptides on viruses with envelope

The peptides antiviral activity was valuated through cytopathic effect on Madin-Darby Bovine Kidney cells (MDBK). In particular NCP-3 was tested against BoHV-1, IBR virus of the Herpesviridae family, subfamily Alphaherpesvirinae, genus Varicellovirus.

### Methods:

### a) Viral inoculum preparation:

MDBK cells were infected with BoHV-1 in DMEM medium with 2% of fetal bovine serum. To obtain a final concentration of 100 TCID50 in 25 µl of DMEM medium, Virus was diluted in DMEM medium without fetal bovine serum.

### b) Serial micro-dilution:

NCP-3 was serially diluted in 25 µl of DMEM medium in microtiter plate wells, to obtain a peptide final concentration between 100 µg/ml (41.6 µM) and 3.125 µg/ml (1.3 µM) after the viral inoculum addition.

### c) Cellular substrate addition:

After 2 hours of incubation of the microtiter plates with NCP-3 and BoHV-1 at room temperature, 50 µl of DMEM medium with 10% of fetal bovine serum and MDBK cells (2x10⁵ cell/ml) in every wells were added. Incubation of about 72 hours at 37°C in humid atmosphere with 5% of CO2 follows. After the incubation, the NCP-3 virus-neutralizing activity was valuated through the virus cytopathic effect on cells using a phase contrast microscope.

### Results:

in the virus neutralization test, NCP-3, from concentration of 25 µg/ml, completely inhibits cytopathic effect due to the BoHV-1 replication.

### Example 8: antimicrobial peptides cytotoxicity

This example tests the antimicrobial peptides cytotoxicity, observing and quantifying haemolysis on mutton red blood cells (RBC) or presence of cells intensely coloured with Trypan Blue vital dye with or without peptide.

### Methods:

### a) Haemolysis test:

Mutton RBCs, sampled immediately before the test, were washed with isotonic buffer to obtain clear supernatant. The washed RBCs were incubated with different peptide concentrations for 1 hour at room temperature. After this the solution was centrifuged and the haemolysis in the supernatant quantified with a spectrophotometer at 450 nm. The positive control was RBC lysed with 0,1% of Tryton. The sample determined the maximal optical density. The haemolysis percentage was calculated as sample optical density at 450 nm and positive control optical density ratio. MHC10 was the minimal peptide concentration able to lyse 10% of RBC.

### b) Peptides cytotoxicity against eukaryotic cells:

Peptides cytotoxicity against epithelial MDBK cells (Madin Darby Bovine Kidney) was determined using cell cultures of about 5x10⁵ cells/ml with and without peptides, with overnight incubation at 37°C with CO2. The negative control was in medium without peptide; positive control was in medium with 0.2% of Triton x100, to completely lyse cells. Cells vitality was assessed adding every well with 20 µl of Trypan Blue, incubating other 5 minutes at 37°C. MCC10 was the minimal peptide concentration with 10% of cytotoxicity.

### Results:

Fig.4 shows tested peptides haemolytic action. All peptides have <10% haemolytic effect at maximum concentration (100 µg/ml. namely NCP-0=46.3 µM. NCP-2=43.2 µM and NCP-3=41.6 µM). Peptides results are better than those of Tachiplesina-1. that induces 15% of haemolysis in RBC at 40 µM.

Tab.4 shows peptides cytotoxicity against MDBK cells. All peptides have <10% cytotoxicity at 100 µg/ml (NCP-0=46.3 µM. NCP-2=43.2 µM and NCP-3=41.6 µM). It confirms little or no peptides cytotoxicity towards eukaryotic cells. Dates are better than those for Tachiplesina-1, that has 10% cell death at a concentration of 20 µM.

**Tab. 4: Cytotoxicity evaluation of NCP-0, NCP-2 and NCP-3. Cell number percentage reduction in comparison with control. Replicated data in three different tests.**

| **Epithelial MDBK cells** | **Peptide concentration** | **Peptide concentration** |
|---|---|---|
| | 100 µg/ml (NCP-0=46.3 µM. NCP-2=43.2 µM. NCP-3=41.6 µM) | 12.5 µg/ml (NCP-0=5.2 µM. NCP-2=5.4 µM. NCP-3=5.2 µM) |
| NCP-0 | 6% | 4% |
| NCP-2 | 5% | 3% |
| NCP-3 | 7% | 3% |

### Example 9: peptides antimicrobial activity in presence of different NaCl percentages

The example verifies the peptides antimicrobial effect in presence of scalar concentration of NaCl.

### Methods:

### a) Serial micro-dilution:

Peptides were tested in the ability to inhibit bacterial growth in liquid medium. 50 µl of peptides at a concentration between 0.2 µM and 50 µM were added in microtiter plate wells with phosphate buffer 10 mM with 125-250 mM of NaCl and one of these bacteria: E. coli ATCC 25922, Pseudomonas aeruginosa ATCC 27853 and Staphylococcus aureus methicillin resistant ATCC 43300. After 2 hours incubation at 37°C, bacteria were plated on solid culture medium. Bacterial cultures were cultivated without peptides as growth control.

### b) Transplant on agar

The bacterial suspension and peptides at differing concentrations were put in contact for about 2 hours at 37°C. After this incubation, 20 µl from every well of the plate were transplanted on solid culture medium, McConkey agar for Gram-negative and Columbia agar with 5% of bovine erythrocytes for Gram-positive bacteria. The inoculum was evenly distributed on the agar medium using a single use sterile handle. After an incubation time of 24 hours at 37°C, the bacterial colonies (cfu) were counted.

### Results:

The following tables show the activity expresed as inhibition percentage of bacterial growth in presence of scalar concentration of NaCl. Every peptide was tested at concentration of 100 µg/ml (NCP-0=46.3 µM; NCP-2=43.2 µM; NCP-3=41.6 µM) and 12.5 µg/ml (NCP-0=5.2 µM; NCP-2=5.4 µM; NCP-3=5.2 µM).

**Tab. 5: Antimicrobial activity (% to control) against E. coli ATCC 25922, Pseudomonas aeruginosa ATCC 27853 and Staphylococcus aureus methicillin resistant ATCC 43300 in presence of 10 mM of NaCl**

| **10 mM** | **E. coli ATCC 25922** | | **Pseudomonas aeruginosa ATCC 27853** | | **MRSA ATCC 43300** | |
|---|---|---|---|---|---|---|
| **Peptide** | **100 µg/ml** | **12.5 µg/ml** | **100 µg/ml** | **12.5 µg/ml** | **100 µg/ml** | **12.5 µg/ml** |
| NCP-0 | 3% | 0% | 2% | 0% | 2% | 0% |
| NCP-2 | 95% | 73% | 98% | 95% | 0% | 0% |
| NCP-3 | 100% | 100% | 100% | 100% | 100% | 100% |

**Tab. 6: Antimicrobial activity (% to control) against E. coli ATCC 25922, Pseudomonas aeruginosa ATCC 27853 and Staphylococcus aureus methicillin resistant ATCC 43300 in presence of 125 mM of NaCl**

| **125 mM** | **E. coli ATCC 25922** | | **Pseudomonas aeruginosa ATCC 27853** | | **MRSA ATCC 43300** | |
|---|---|---|---|---|---|---|
| **Peptide** | **100 µg/ml** | **12.5 µg/ml** | **100 µg/ml** | **12.5 µg/ml** | **100 µg/ml** | **12.5 µg/ml** |
| NCP-0 | 0% | 0% | 0% | 0% | 0% | 0% |
| NCP-2 | 70% | 51% | 90% | 86% | 0% | 0% |
| NCP-3 | 100% | 100% | 100% | 100% | 100% | 96.75% |

**Tab. 7: Antimicrobial activity (% to control) against E. coli ATCC 25922, Pseudomonas aeruginosa ATCC 27853 and Staphylococcus aureus methicillin resistant ATCC 43300 in presence of 250 mM of NaCl**

| **250 mM** | **E. coli ATCC 25922** | | **Pseudomonas aeruginosa ATCC 27853** | | **MRSA ATCC 43300** | |
|---|---|---|---|---|---|---|
| **Peptide** | **100 µg/ml** | **12.5 µg/ml** | **100 µg/ml** | **12.5 µg/ml** | **100 µg/ml** | **12.5 µg/ml** |
| NCP-0 | 0% | 0% | 0% | 0% | 0% | 0% |
| NCP-2 | 680% | 43% | 78% | 65% | 0% | 0% |
| NCP-3 | 100% | 96.75% | 100% | 100% | 100% | 37.5% |

In reference to Tab.5, results with NaCl 10 mM agree with those of example 3. With NaCl 125 mM (Tab.6), NCP-3 maintains the maximal antimicrobial activity and NCP-2 shows a good activity against Gram-negative bacteria. With NaCl 250 mM (Tab.7), NCP-3 maintains excellent activity against all tested bacteria, and NCP-2 as a good antimicrobial activity against Gram-negative bacteria, despite the high salt concentration (achievable only in a few pathological states like pulmonary fluid during cystic fibrosis).

### Example 10: peptides antimicrobial activity in presence of Muller-Hinton 20%

This example verifies the peptides antimicrobial activity in presence of a concentration of 20% of Muller-Hinton liquid medium, characterized by the presence of salts with high concentration. This concentration is the minimal concentration of the medium that allows to have a bacterial growth comparable to the growth control.

Peptides were tested in the ability to inhibit bacterial growth in liquid medium. 50 µl of peptides at a concentration between 3.1 µg/ml (NCP-0=1.4µM; NCP-2=1.8 µM; NCP-3=1.3 µM) and 100 µg/ml (NCP-0=46.3 µM; NCP-2=43.2 µM; NCP-3=41.6 µM) were added in microtiter plate wells with phosphate buffer 10 mM with Muller-Hinton liquid medium al the final concentration of 20% and one of these bacteria: E. coli ATCC 25922, Pseudomonas aeruginosa ATCC 27853, Staphylococcus aureus ATCC 25923 and Staphylococcus aureus methicillin resistant ATCC 43300. After 2 hours incubation at 37°C, bacteria were transplanted on solid culture medium. Bacterial cultures were cultivated without peptides as growth control.

### Results:

data on tables below show that, unlike the reference peptide NCP-0, NCP-2 and NCP-3 have a good antimicrobial activity against all tested bacteria. Peptides structural characteristics (in particular distribution of charge and hydrophobic regions) allow to maintain the antimicrobial activity, even in unfavorable environment.

**Tab. 8: Antimicrobial activity (% to control) against E. coli ATCC 25922, in presence of 20% of Muller-Hinton medium. NCP-3 (PB) referred to the test in phosphate buffer without MH medium**

| | **E. coli ATCC 25922** | | | |
|---|---|---|---|---|
| | **100 µg/ml** | **12.5 µg/ml** | **6.3 µg/ml** | **3.1 µg/ml** |
| NCP-0 | 0% | 0% | 0% | 0% |
| NCP-2 | 100% | 100% | 99.1% | 91% |
| NCP-3 | 100% | 100% | 100% | 100% |
| NCP-3 (PB) | 100% | 100% | 100% | 100% |

**Tab. 9: Antimicrobial activity (% to control) against Pseudomonas aeruginosa ATCC 27853, in presence of 20% of Muller-Hinton medium. NCP-3 (PB) referred to the test in phosphate buffer without MH medium**

| | **Pseudomonas aeruginosa ATCC 27853** | | | |
|---|---|---|---|---|
| | **100 µg/ml** | **12.5 µg/ml** | **6.3 µg/ml** | **3.1 µg/ml** |
| NCP-0 | 0% | 0% | 0% | 0% |
| NCP-2 | 86.7% | 0% | 0% | 0% |
| NCP-3 | 100% | 100% | 98% | 66.7% |
| NCP-3 (PB) | 100% | 100% | 98% | 93.8% |

**Tab. 10: Antimicrobial activity (% to control) against Staphylococcus aureus ATCC 25923, in presence of 20% of Muller-Hinton medium. NCP-3 (PB) referred to the test in phosphate buffer without MH medium**

| | **Staphylococcus aureus ATCC 25923** | | | |
|---|---|---|---|---|
| | **100 µg/ml** | **12.5 µg/ml** | **6.3 µg/ml** | **3.1 µg/ml** |
| NCP-0 | 0% | 0% | 0% | 0% |
| NCP-2 | 0% | 0% | 0% | 0% |
| NCP-3 | 100% | 100% | 94.3% | 58.3% |
| NCP-3 (PB) | 100% | 100% | 100% | 98.3% |

**Tab. 11: Antimicrobial activity (% to control) against MRSA ATCC 43300, in presence of 20% of Muller-Hinton medium. NCP-3 (PB) referred to the test in phosphate buffer without MH medium**

| | **MRSA ATCC 43300** | | | |
|---|---|---|---|---|
| | **100 µg/ml** | **12.5 µg/ml** | **6.3 µg/ml** | **3.1 µg/ml** |
| NCP-0 | 0% | 0% | 0% | 0% |
| NCP-2 | 0% | 0% | 0% | 0% |
| NCP-3 | 100% | 97% | 50% | 0% |
| NCP-3 (PB) | 100% | 100% | 100% | 82.8% |

## Claims

1. Peptides having structure **A-B-C-D-E-F-G** wherein:
- **A** represents a basic aminoacid,
- **B, D, F** represent respectively 5, 3 and 3 aminoacids, chosen in the group of hydrophobic aminoacids, wherein at least one of the conditions (i)-(iii) is met:
(i) **B** is chosen among LIPIL, LIWIL, LIPIA, LIFIL, LILIL, LIYIL, or any combination of hydrophobic aminoacids containing W;
(ii) **D** represents IPA;
(iii) **F** is chosen among LFY, LIY, LLY, LVY;
- **C, E** represent 3 aminoacids, wherein each C and E consist of at least 1 basic aminoacid, at least 1 aminoacid forming hydrogen bonds and, optionally, one or more aminoacids chosen from G, L and I.
- **G** represents 2 aminoacids, chosen among basic aminoacids and/or hydrophobic aminoacids, salts and/or mixtures thereof.

2. Peptides according to claim 1, wherein **C** represents SKT.

3. Peptides according to claim 1, wherein **C** represents SKT, **A** represents K.

4. Peptides according to claim 1, wherein **C** represents SKT, **A** represents K, **G** represents KI.

5. Peptides according to claim 1, wherein **C** represents SKT, **A** represents K, **G** represents KI, **E** is chosen among IKN and GKN.

6. Peptides according to any of claims 1-5, wherein at least two of the conditions (i), (ii), (iii) are met.

7. Peptides according to claim 6, wherein all three conditions (i), (ii), (iii) are met.

8. Peptides according to any of the preceding claims, in accordance with one of the following conditions (i)- (ix):
(i) **A** is K, **B** is LIPIL, **C** is SKT, **D** is IPA, **E** IKN, **F** is LFY, **G** is KI, or
(ii) **A** is K, **B** is LIWIL, **C** is SKT, **D** is IPA, **E** is IKN, **F** is LFY, **G** is KI, or
(iii) **A** is K, **B** is LIPIA, **C** is SKT, **D** is IPA, **E** is GKN, **F** is LFY, G is KI, or
(iv) **A** is K, **B** LIPIA, **C** is SKT, **D** is IPA, **E** is GKN, **F** is LIY, **G** is KI, or
(v) **A** is K, **B** is LIPIA, **C** is SKT, **D** is IPA, **E** is GKN, **F** is LLY, **G** is KI, or,
(vi) **A** is K, **B** is LIPIA, **C** is SKT, **D** is IPA, **E** is GKN, **F** is LVY, **G** is KI, or
(vii) **A** is K, **B** is LIFIL, **C** is SKT, **D** is IPA, **E** is IKN, **F** is LFY, **G** is KI, or
(viii) **A** is K, **B** is LILIL, **C** is SKT, **D** is IPA, **E** is IKN, **F** is LFY, **G** is KI, or
(ix) **A** is K, **B** is LIYIL, **C** is SKT, **D** is IPA, **E** is IKN, **F** is LFY, **G** is KI.

9. Process for synthesizing a peptide according to any of claims 1-8, comprising adding in sequence the aminoacids necessary to form said structure A-B-C-D-E-F-G.

10. Pharmaceutical composition comprising one or more peptides as described in claims 1-8.

11. Peptides as described in claims 1-8, or pharmaceutical composition thereof according to claim 10, for use in the treatment or prevention of pathologies having bacterial, fungal and/or viral origin.

12. Peptides or composition according to claim 11, wherein said pathologies involve a concentration of NaCl in the organism higher than physiologic levels.

## Patentansprüche

1. Peptide mit der Struktur **A-B-C-D-E-F-G,** wobei:
- **A** für eine basische Aminosäure steht,
- **B, D** und **F** für jeweils 5, 3 und 3 Aminosäuren aus der Gruppe der hydrophoben Aminosäuren stehen, wobei mindestens eine der folgenden Bedingungen (i)-(iii) erfüllt ist:
(i) **B** ist ausgewählt aus LIPIL, LIWIL, LIPIA, LIFIL, LILIL, LIYIL oder einer beliebigen Kombination von hydrophoben Aminosäuren, die W enthalten;
(ii) **D** steht für IPA;
(iii) **F** ist ausgewählt aus LFY, LIY, LLY, LVY;
- **C** und **E** für 3 Aminosäuren stehen, wobei jedes C und E aus mindestens 1 basischen Aminosäure, mindestens 1 Wasserstoffbrückenbindungen bildenden Aminosäure und, optional, einer oder mehreren Aminosäuren aus G, L und I besteht;
- **G** für 2 Aminosäuren steht, die aus basischen Aminosäuren und/oder hydrophoben Aminosäuren, Salzen und/oder Mischungen davon ausgewählt sind.

2. Peptide nach Anspruch 1 wobei **C** für SKT steht.

3. Peptide nach Anspruch 1, wobei **C** für SKT steht, A für **K** steht.

4. Peptide nach Anspruch 1, wobei **C** für SKT steht, **A** für K steht, **G** für KI steht.

5. Peptide nach Anspruch 1, wobei **C** für SKT steht, **A** für K steht, **G** für KI steht, **E** aus IKN und GKN ausgewählt ist.

6. Peptide nach einem der Ansprüche 1-5, wobei mindestens zwei der Bedingungen (i), (ii) und (iii) erfüllt sind.

7. Peptide nach Anspruch 6, wobei alle drei Bedingungen (i), (ii) und (iii) erfüllt sind.

8. Peptide nach einem der vorhergehenden Ansprüche, gemäß einer der folgenden Bedingungen (i)-(ix):
(i) **A** ist K, **B** ist LIPIL, **C** ist SKT, **D** ist IPA, **E** ist IKN, **F** ist LFY, **G** ist KI, oder
(ii) **A** ist K, **B** ist LIWIL, **C** ist SKT, **D** ist IPA, **E** ist IKN, **F** ist LFY, **G** ist KI, oder
(iii) **A** ist K, **B** ist LIPIA, **C** ist SKT, **D** ist IPA, **E** ist GKN, **F** ist LFY, **G** ist KI, oder
(iv) **A** ist K, **B** ist LIPIA, **C** ist SKT, **D** ist IPA, **E** ist GKN, **F** ist LIY, **G** ist KI, oder
(v) **A** ist K, **B** ist LIPIA, **C** ist SKT, **D** ist IPA, **E** ist GKN, **F** ist LLY, **G** ist KI, oder
(vi) **A** ist K, **B** ist LIPIA, **C** ist SKT, **D** ist IPA, **E** ist GKN, **F** ist LVY, **G** ist KI, oder
(vii) **A** ist K, **B** ist LIFIL, **C** ist SKT, **D** ist IPA, **E** ist IKN, **F** ist LFY, **G** ist KI, oder
(viii) **A** ist K, **B** ist LILIL, **C** ist SKT, **D** ist IPA, **E** ist IKN, **F** ist LFY, **G** ist KI, oder
(ix) **A** ist K, **B** ist LIYIL, **C** ist SKT, **D** ist IPA, **E** ist IKN, **F** ist LFY, **G** ist KI.

9. Verfahren zur Synthese eines Peptids nach einem der Ansprüche 1-8, welches das sequenzielle Zugeben der Aminosäuren, die zur Bildung der Struktur A-B-C-D-E-F-G erforderlich sind, umfasst.

10. Pharmazeutische Zusammensetzung, welche ein oder mehrere Peptide nach den Ansprüchen 1-8 umfasst.

11. Peptide nach den Ansprüchen 1-8 oder pharmazeutische Zusammensetzung davon nach Anspruch 10 zur Verwendung in der Behandlung oder Prävention von Pathologien bakteriellen, fungalen und/oder viralen Ursprungs.

12. Peptide oder Zusammensetzung nach Anspruch 11, wobei die Pathologien eine NaCl-Konzentration im Organismus, welche höher ist als physiologische Mengen, mit sich bringen.

## Revendications

1. Peptides ayant la structure **A-B-C-D-E-F-G** dans laquelle:
- **A** représente un acide aminé basique,
- **B, D,** F représentent respectivement 5, 3 et 3 acides aminés, sélectionnés dans le groupe des acides aminés hydrophobes, dans lesquels au moins l'une des conditions (i) à (iii) est remplie:
(i) **B** est sélectionné parmi LIPIL, LIWIL, LIPIA, LIFIL, LILIL, LIYIL, ou toute combinaison d'acides aminés hydrophobes contenant W;
(ii) D représente IPA;
(iii) **F** est sélectionné parmi LFY, LIY, LLY, LVY;
- **C, E** représentent 3 acides aminés, dans lesquels chaque C et E sont constitués par au moins 1 acide aminé basique, au moins 1 acide aminé formant des liaisons hydrogène et, éventuellement, un ou plusieurs acides aminés sélectionnés parmi G, L et I.
- **G** représente 2 acides aminés, sélectionnés parmi les acides aminés basiques et/ou les acides aminés hydrophobes, leurs sels et/ou leurs mélanges.

2. . Peptides selon la revendication 1, dans lesquels **C** représente SKT.

3. . Peptides selon la revendication 1, dans lesquels **C** représente SKT, **A** représente K.

4. . Peptides selon la revendication 1, dans lesquels **C** représente SKT, **A** représente K, **G** représente KI.

5. . Peptides selon la revendication 1, dans lesquels **C** représente SKT, **A** représente K, **G** représente KI, **E** est sélectionné parmi IKN et GKN.

6. . Peptides selon l'une quelconque des revendications 1 à 5, dans lesquels au moins deux des conditions (i), (ii), (iii) sont remplies.

7. . Peptides selon la revendication 6, dans lesquels les trois conditions (i), (ii), (iii) sont remplies.

8. . Peptides selon l'une quelconque des revendications précédentes, conformément à l'une des conditions (i) à (ix) suivantes:
(i) **A** est K, **B** est LIPIL, **C** est SKT, **D** est IPA, **E** IKN, **F** est LFY, **G** est KI, ou
(ii) **A** est K, **B** est LIWIL, **C** est SKT, **D** est IPA, **E** est IKN, **F** est LFY, **G** est KI, ou
(iii) **A** est K, **B** est LIPIA, **C** est SKT, **D** est IPA, **E** est GKN, **F** est LFY, **G** est KI, ou
(iv) **A** est K, **B** LIPIA, **C** est SKT, **D** est IPA, **E** est GKN, **F** est LIY, **G** est KI, ou
(v) **A** est K, **B** est LIPIA, **C** est SKT, **D** est IPA, **E** est GKN, **F** est LLY, **G** est KI, ou,
(vi) **A** est K, **B** est LIPIA, **C** est SKT, **D** est IPA, **E** est GKN, **F** est LVY, **G** est KI, ou
(vii) **A** est K, **B** est LIFIL, **C** est SKT, **D** est IPA, **E** est IKN, **F** est LFY, **G** est KI, ou
(viii) **A** est K, **B** est LILIL, **C** est SKT, **D** est IPA, **E** est IKN, **F** est LFY, **G** est KI, ou
(ix) **A** est K, **B** est LIYIL, **C** est SKT, **D** est IPA, **E** est IKN, **F** est LFY, **G** est KI.

9. . Procédé pour synthétiser un peptide selon l'une quelconque des revendications 1 à 8, comprenant l'ajout en séquence des acides aminés nécessaires pour former ladite structure A-B-C-D-E-F-G.

10. . Composition pharmaceutique comprenant un ou plusieurs peptides tels que décrits dans les revendications 1 à 8.

11. . Peptides tels que décrits dans les revendications 1 à 8, ou leur composition pharmaceutique selon la revendication 10, pour une utilisation dans le traitement ou la prévention de pathologies ayant une origine bactérienne, fongique et/ou virale.

12. . Peptides ou composition selon la revendication 11, dans lesquels lesdites pathologies impliquent une concentration de NaCl dans l'organisme supérieure aux niveaux physiologiques.
